# EUROPEAN PATENT APPLICATION

(11) **EP 4 628 584 A1**
(43) Date of publication of application: **08.10.2025**
(21) Application number: 24168265.7
(22) Date of filing: 03.04.2024
(51) Int. Cl.: C12N 15/113, A61K 48/00, C07K 4/00

(54) **Y CHROSOMAL GENES FOR THE TREATMENT OR DIAGNOSIS OF CARDIOVASCULAR DISEASE**

(71) Applicant: Johann-Wolfgang-Goethe-Universität Frankfurt am Main, 60323 Frankfurt am Main (DE)
(72) Inventor: Dimmeler, Stefanie, 60594 Frankfurt am Main (DE); Zanders, Lukas, 60385 Frankfurt am Main (DE); Fleck, Fenja, 60594 Frankfurt am Main (DE); Shumliakivska, Mariana, 60528 Frankfurt am Main (DE); Zeiher, Andreas, 60594 Frankfurt am Main (DE)
(74) Representative: Kuttenkeuler, David

(57) **Abstract**

[1] The invention is based on the identification of Y chromosomal genes responsible for the pathophysiology of cardiovascular diseases. The invention provides methods for the identification of compounds useful in the treatment of cardiovascular diseases as well as methods, compounds, and compositions useful for the treatment or prevention of cardiovascular diseases and/or risks.

## Description

### FIELD OF THE INVENTION

The invention is based on the identification of Y chromosomal genes responsible for the pathophysiology of cardiovascular diseases. The invention provides methods for the identification of compounds useful in the treatment of cardiovascular diseases as well as methods, compounds, and compositions useful for the treatment or prevention of cardiovascular diseases and/or risks.

### DESCRIPTION

Higher age is the strongest cardiovascular risk factor, which to date is considered to be non-modifiable. While the phenotypes of cardiovascular aging and age-associated diseases are well-established, the regulating factors remain poorly understood [1]. As life expectancy is rising, elucidating the mechanisms underlying age-associated diseases poses a key task in cardiovascular medicine [2].

Moreover, substantial sex differences in the incidence, clinical presentation and outcomes are typical characteristics of cardiovascular disease, with men more often developing atherosclerotic disease and women being more prone to develop heart failure with preserved ejection fraction. While previous research has focused on hormonal aspects, recent data suggests additional genetic aspects involved in sex differences in cardiovascular disease [3].

Over the last ten years, loss of the Y chromosome (LoY) in peripheral blood mononuclear cells (PBMCs) has emerged as a new cardiovascular risk factor [4, 5]. The chromosome loss occurs at a post-zygotic stage, resulting in a mosaicism with inter-individually varying frequencies of Y-chromosome deficient PBMCs. Mosaic loss of Y (mLoY) is strongly age-associated, affecting about 2.5% of men at age 40 and more than 50% above the age of 90, hence representing the most common genetic aberration in men [6-8]. Early population-wide studies showed an increased all-cause, cancer-associated and cardiovascular mortality, as well as an increase in atherosclerotic disease [4]. More recent evidence indicates worse prognosis in patients with mLoY after carotid endarterectomy for carotid artery stenosis and transcatheter aortic valve replacement for severe aortic valve stenosis [5, 9].

A causal role of mLoY in cardiovascular disease was shown in a recent study, in which competitive transplantation of wild-type and LOY hematopoietic stem cells resulted in increased interstitial myocardial fibrosis, decreased systolic function and a shorter overall lifespan. Moreover, upon left ventricular pressure overload, mLOY induces pronounced interstitial myocardial fibrosis, which was rescued after systemic treatment with a TGFβ neutralizing antibody [10]. A pro-fibrotic transcriptomic signature was also found in LOY peripheral blood mononuclear cells (PBMCs) of patients with severe aortic valve stenosis [9].

It remains unknown, through which mechanism mLOY in peripheral blood cells exerts its effects on the cardiovascular system, especially if the loss of a single or of the combination of several Y-chromosomal genes are responsible for the observed effects. Interestingly, the Y-chromosomal haplotype I is associated with an increased risk for coronary artery disease and a downregulation of the Y-chromosomal genes UTY and PRKY, potentially indicating a role for single genes in this setting [11, 12].

Thus, it is an object of the invention to identify factors responsible for the cardiovascular effects and to provide new treatment strategies to tackle cardiovascular adverse events and diseases. It is further an objective of the invention to provide options for the identification of therapeutic compounds useful in the treatment of such disorders.

### BRIEF DESCRIPTION OF THE INVENTION

Generally, and by way of brief description, the main aspects of the present invention can be described as follows:

In **a first aspect,** the invention pertains to a compound or composition for use in the treatment of a cardiovascular disease in a subject, wherein the compound or composition is, or comprises, and agonist of expression, function and/or stability of a gene, or protein product of a gene, selected from *Ribosomal Protein S4 Y-Linked 1* (*RPS4Y1*), *DEAD-Box Helicase 3 Y-linked (DDX3Y), Eukaryotic Translation Initiation Factor 1A Y-linked* (*EIF1AY*)*, Ubiquitously Transcribed Tetratricopeptide Repeat Containing Y-linked (UTY), Zink Finger Protein Y-linked* (*ZFY*), *Lysine Demethylase 5D (KDM5D), Ubiquitin Specific Peptidase 9 Y-linked (USP9Y)* and the non-coding genes *Testis Expressed Transcript Y-linked 14 (TTTY14)* and *Long Intergenic Non-Coding RNA 278 (LINC00278).*

In **a second aspect,** the invention pertains to a method for identifying a modulator cell-mediated regulation of myocardial cell function, the method comprising the steps:
- Providing a first cell and a second cell, wherein the second cell is a myocardial cell;
- Culturing the first cell to obtain a test first cell under conditions which are selected from:
   (i) where the expression one or more known candidate genes are being perturbed, preferably knocked out or knocked in, in the first cell:
   (ii) With one or more genetic expression constructs being expressed in the first cell to overexpress, or ectopically express, one or more known candidate genes in the first cell; and
   (iii) Where the first cell is cultured in contact with one or more candidate compounds
- optionally culturing a control first cell under control conditions (not the conditions of step (b));
- bringing into contact the second cell and the test first cell, or the second cell and conditioned medium of the culture of the test first cell, and, optionally, culturing the second cell under the contact conditions:
Wherein a difference of the test first cell to a control or control first cell in one or more markers of myocardial function indicates that the one or more known candidate genes or compounds is a modulator of a cell mediated regulation of myocardial cell function.

In **a third aspect,** the invention pertains to a method for the identification of a compound useful in the treatment or prevention of a cardiovascular disorder, the method comprising the step of:
- Providing a candidate compound, composition, or a candidate medical procedure;
- Providing a test cell or test tissue, wherein the test cell or test tissue is involved with or is associated with the cardiovascular disease;
- Contacting the test cell or test tissue with the candidate compound, composition or performing on the test cell or test tissue the candidate medical procedure;
- Determining in the test cell or test tissue a test gene expression level, or a level of protein, wherein the protein is expressed by such test gene, the test gene being selected from the group consisting of *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDM5D), Ubiquitin Specific Peptidase 9 Y-linked (USP9Y) and the non-coding genes Testis Expressed Transcript Y-linked 14 (TTTY14)* and *Long Intergenic Non-Coding RNA 278 (LINC00278);*
Wherein an increased gene expression level, or an increased protein level in the test cell or test tissue compared to a reference, or compared to a control cell or control tissue not contacted with the candidate compound or composition or medical procedure, indicates that the respective candidate compound or composition or candidate medical procedure is useful in the treatment or prevention of a cardiovascular disease.

In **a fourth aspect,** the invention pertains to a method for diagnosing a subject, the method comprising determining in a biological sample of the subject the level of gene or protein expression of at least one biomarker selected from *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDM5D), Ubiquitin Specific Peptidase 9 Y-linked (USP9Y) and the non-coding genes Testis Expressed Transcript Y-linked 14 (TTTY14)* and *Long Intergenic Non-Coding RNA 278 (LINC00278);* wherein a reduced gene or protein expression indicates an increased risk of cardiovascular disease in the subject.

In **a fifth aspect,** the invention pertains to a use of a biomarker for the diagnosis of an increased risk of cardiovascular disease in a subject, wherein the protein expression or gene expression is selected from an expression of the genes *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDMSD), Ubiquitin Specific Peptidase 9 Y-linked (USP9Y) and the non-coding genes Testis Expressed Transcript Y-linked 14 (TTTY14)* or *Long Intergenic Non-Coding RNA 278 (LINC00278).*

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The present invention relates in its various aspects and embodiments to the expression of RNA or Protein of certain genes located on the human Y chromosomes. The respective gene sequences, as well as RNA products expressed by the genes, and protein or peptide products translated from such RNA, shall be fully included in the present disclosure. All genes are known to the skilled artisan and their sequences and gene products, either RNA or protein, is as information included in various databases such as the HUGO gene nomenclarure database, the NCBI gene database, ENSEMBLE or UniProt. In the following for each of the candidate genes identified in context of the present invention their respective accession numbers of a selection of databases is provided to facilitate sequence identification. Certain sequences are also included in this disclosure directly in the attached sequence protocol which forms part of the present description.

For the purpose of understanding the herein disclosed invention, the following explanations and definitions are provided.

A disease or disorder is "alleviated" or "treated" if the severity of a symptom of the disease, condition, or disorder, or the frequency at which such a symptom is experienced by a subject, or both, are reduced. As used herein, the term "and/or" when used in the context of a list of entities, refers to the entities being present singly or in combination. Thus, for example, the phrase "A, B, C, and/or D" includes A, B, C, and D individually, but also includes any and all combinations and subcombinations of A, B, C, and D.

As use herein, the terms "administration of' and/or "administering" a compound should be understood to refer to providing a compound of the presently disclosed subject matter to a subject in need of treatment.

The term "adult" as used herein, is meant to refer to any non-embryonic or nonjuvenile subject. For example, the term "adult adipose tissue stem cell," refers to an adipose stem cell, other than that obtained from an embryo or juvenile subject.

As used herein, an "agonist" is a composition of matter which, when administered to a mammal such as a human, enhances or extends a biological activity attributable to the level or presence of a target compound or molecule of interest in the mammal.

An "antagonist" is a composition of matter which when administered to a mammal such as a human, inhibits a biological activity attributable to the level or presence of a compound or molecule of interest in the mammal.

As used herein, the term "antisense oligonucleotide" or antisense nucleic acid means a nucleic acid polymer, at least a portion of which is complementary to a nucleic acid which is present in a normal cell or in an affected cell. "Antisense" refers particularly to the nucleic acid sequence of the non-coding strand of a double stranded DNA molecule encoding a protein, or to a sequence which is substantially homologous to the non-coding strand. As defined herein, an antisense sequence is complementary to the sequence of a double stranded DNA molecule encoding a protein. It is not necessary that the antisense sequence be complementary solely to the coding portion of the coding strand of the DNA molecule. The antisense sequence may be complementary to regulatory sequences specified on the coding strand of a DNA molecule encoding a protein, which regulatory sequences control expression of the coding sequences. The antisense oligonucleotides of the presently disclosed subject matter include, but are not limited to, phosphorothioate oligonucleotides and other modifications of oligonucleotides.

An "aptamer" is a compound that is selected in vitro to bind preferentially to another compound (for example, the identified proteins herein). Often, aptamers are nucleic acids or peptides because random sequences can be readily generated from nucleotides or amino acids (both naturally occurring or synthetically made) in large numbers but of course they need not be limited to these.

As used herein, the terms "biologically active fragment" and "bioactive fragment" of a peptide encompass natural and synthetic portions of a longer peptide or protein that are capable of specific binding to their natural ligand and/or of performing a desired function of a protein, for example, a fragment of a protein of larger peptide which still contains the epitope of interest and is immunogenic.

The term "biological sample," as used herein, refers to samples obtained from a subject, including but not limited to skin, hair, tissue, blood, plasma, cells, sweat, and urine. Preferred biological samples contain blood cells, or precursor cells of blood cells.

The term "cardiovascular disease" may include e.g. coronary artery disease (also known as coronary heart disease and ischemic heart disease), peripheral artery disease (e.g. peripheral endothelial dysfunction), renal artery stenosis, and aortic aneurysm, atherosclerotic plaque formation. There are also many cardiovascular diseases that involve the heart, e.g. cardiomyopathy, hypertensive heart disease, heart failure, pulmonary heart disease, cardiac arrhythmias, endocarditis, inflammatory cardiomyopathy, myocarditis, toxic, and autoimmune disorders, eosinophilic myocarditis, valvular heart disease, congenital heart disease, and rheumatic heart disease. Preferably, the cardiovascular disease is selected from hypertrophic heart disease, valvular heart disease, atherosclerotic plaque formation and coronary artery disease.

A "compound," as used herein, refers to a polypeptide, an isolated nucleic acid, or other agent used in the method of the presently disclosed subject matter.

A "control" cell, tissue, sample, or subject is a cell, tissue, sample, or subject of the same type as a test cell, tissue, sample, or subject. The control may, for example, be examined at precisely or nearly the same time the test cell, tissue, sample, or subject is examined. The control may also, for example, be examined at a time distant from the time at which the test cell, tissue, sample, or subject is examined, and the results of the examination of the control may be recorded so that the recorded results may be compared with results obtained by examination of a test cell, tissue, sample, or subject. The control may also be obtained from another source or similar source other than the test group or a test subject, where the test sample is obtained from a subject suspected of having a condition, disease, or disorder for which the test is being performed.

A "test" cell is a cell being examined.

As used herein, the terms "condition", "disease condition", "disease", "disease state", and "disorder" refer to physiological states in which diseased cells or cells of interest can be targeted with the compositions of the presently disclosed subject matter. In some embodiments, a disease is a cardiovascular disease, and may be specified in more detail elsewhere herein.

As used herein, the term "diagnosis" refers to detecting a risk or propensity to a condition, disease, or disorder. In any method of diagnosis exist false positives and false negatives. Any one method of diagnosis does not provide 100% accuracy.

A "disease" is a state of health of an animal wherein the animal cannot maintain homeostasis, and wherein if the disease is not ameliorated then the animal's health continues to deteriorate.

As used herein, an "effective amount" or "therapeutically effective amount" refers to an amount of a compound or composition sufficient to produce a selected effect, such as but not limited to alleviating symptoms of a condition, disease, or disorder. In the context of administering compounds in the form of a combination, such as multiple compounds, the amount of each compound, when administered in combination with one or more other compounds, may be different from when that compound is administered alone. Thus, an effective amount of a combination of compounds refers collectively to the combination as a whole, although the actual amounts of each compound may vary. The term "more effective" means that the selected effect occurs to a greater extent by one treatment relative to the second treatment to which it is being compared.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA, and mRNA) or a defined sequence of amino acids and the biological properties resulting therefrom. Thus, a gene encodes a protein if transcription and translation of an mRNA corresponding to or derived from that gene produces the protein in a cell or other biological system and/or an in vitro or ex vivo system. Both the coding strand, the nucleotide sequence of which is identical to the mRNA sequence (with the exception of uracil bases presented in the latter) and is usually provided in Sequence Listing, and the non-coding strand, used as the template for transcription of a gene or cDNA, can be referred to as encoding the protein or other product of that gene or cDNA.

The term "expression construct," as used herein, refers to a nucleic acid construct comprising nucleic elements sufficient for the expression of a gene product of any of the genese listed herein. The expression construct may express an mRNA (for protein expression) or a non-coding RNA (IncRNA). Typically, an expression construct comprises a nucleic acid encoding a gene product (e.g., transgene) operatively linked to a promoter sequence. The term "operatively linked" refers to the association of two or more nucleic acid fragments on a single nucleic acid fragment so that the function of one is affected by the other. For example, a promoter is operatively linked with a coding sequence when it is capable of affecting the expression of that coding sequence ( e.g ., the coding sequence is under the transcriptional control of the promoter). Encoding sequences can be operatively linked to regulatory sequences in sense or antisense orientation. In some embodiments, the promoter is a heterologous promoter.

A "fragment", "segment", or "subsequence" is a portion of an amino acid sequence, comprising at least one amino acid, or a portion of a nucleic acid sequence comprising at least one nucleotide. The terms "fragment," "segment", and "subsequence" are used interchangeably herein.

As used herein, the term "fragment", as applied to a protein or peptide, can ordinarily be at least about 3-15 amino acids in length, at least about 15-25 amino acids, at least about 25-50 amino acids in length, at least about 50-75 amino acids in length, at least about 75- 100 amino acids in length, and greater than 100 amino acids in length.

As used herein, the term "fragment" as applied to a nucleic acid, may ordinarily be at least about 20 nucleotides in length, typically, at least about 50 nucleotides, more typically, from about 50 to about 100 nucleotides, in some embodiments, at least about 100 to about 200 nucleotides, in some embodiments, at least about 200 nucleotides to about 300 nucleotides, yet in some embodiments, at least about 300 to about 350, in some embodiments, at least about 350 nucleotides to about 500 nucleotides, yet in some embodiments, at least about 500 to about 600, in some embodiments, at least about 600 nucleotides to about 620 nucleotides, yet in some embodiments, at least about 620 to about 650, and most in some embodiments, the nucleic acid fragment will be greater than about 650 nucleotides in length. In the case of a shorter sequence, fragments are shorter.

The determination of percent identity between two nucleotide or amino acid sequences can be accomplished using a mathematical algorithm. For example, a mathematical algorithm useful for comparing two sequences is the algorithm of Karlin & Altschul, 1990a, modified as in Karlin &Altschul, 1993). This algorithm is incorporated into the NBLAST and XBLAST programs of Altschul et al., 1990a, and can be accessed, for example at the National Center for Biotechnology Information (NCBI) world wide web site. BLAST nucleotide searches can be performed with the NBLAST program (designated "blastn" at the NCBI web site), using the following parameters: gap penalty = 5; gap extension penalty = 2; mismatch penalty = 3; match reward = 1; expectation value 10.0; and word size = 11 to obtain nucleotide sequences homologous to a nucleic acid described herein. BLAST protein searches can be performed with the XBLAST program (designated "blastn" at the NCBI web site) or the NCBI "blastp" program, using the following parameters: expectation value 10.0, BLOSUM62 scoring matrix to obtain amino acid sequences homologous to a protein molecule described herein. To obtain gapped alignments for comparison purposes, Gapped BLAST can be utilized as described in Altschul et al., 1997. Alternatively, PSI-Blast or PHI-Blast can be used to perform an iterated search which detects distant relationships between molecules (Altschul et al., 1997) and relationships between molecules which share a common pattern. When utilizing BLAST, Gapped BLAST, PSI-Blast, and PHI-Blast programs, the default parameters of the respective programs (e.g., XBLAST and NBLAST) can be used.

The percent identity between two sequences can be determined using techniques similar to those described above, with or without allowing gaps. In calculating percent identity, typically exact matches are counted.

The terms "measuring the level of expression" and "determining the level of expression" as used herein refer to any measure or assay which can be used to correlate the results of the assay with the level of expression of a gene or protein of interest. Such assays include measuring the level of mRNA, protein levels, etc. and can be performed by assays such as northern and western blot analyses, binding assays, immunoblots, etc. The level of expression can include rates of expression and can be measured in terms of the actual amount of an mRNA or protein present. Such assays are coupled with processes or systems to store and process information and to help quantify levels, signals, etc. and to digitize the information for use in comparing levels.

The term "modulate", as used herein, refers to changing the level of an activity, function, or process. The term "modulate" encompasses both inhibiting and stimulating an activity, function, or process. The term "modulate" is used interchangeably with the term "regulate" herein.

"Polypeptide" refers to a polymer composed of amino acid residues, related naturally occurring structural variants, and synthetic non-naturally occurring analogs thereof linked via peptide bonds, related naturally occurring structural variants, and synthetic non-naturally occurring analogs thereof.

"Synthetic peptides or polypeptides" refers to non-naturally occurring peptides or polypeptides. Synthetic peptides or polypeptides can be synthesized, for example, using an automated polypeptide synthesizer. Various solid phase peptide synthesis methods are known to those of skill in the art.

The term "prevent," as used herein, means to stop something from happening, or taking advance measures against something possible or probable from happening. In the context of medicine, "prevention" generally refers to action taken to decrease the chance of getting a disease or condition or a complication thereof. It is noted that "prevention" need not be absolute, and thus can occur as a matter of degree.

A "preventive" or "prophylactic" treatment is a treatment administered to a subject who does not exhibit signs, or exhibits only early signs, of a condition, disease, disorder or a complication thereof. A prophylactic or preventative treatment is administered for the purpose of decreasing the risk of developing pathology associated with developing the condition, disease, disorder or a complication thereof.

As used herein, the term "promoter/regulatory sequence" means a nucleic acid sequence which is required for expression of a gene product operably linked to the promoter/regulator sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a promoter which drives expression of a gene to which it is operably linked, in a constant manner in a cell. By way of example, promoters which drive expression of cellular housekeeping genes are considered to be constitutive promoters.

An "inducible" promoter is a nucleotide sequence which, when operably linked with a polynucleotide which encodes or specifies a gene product, causes the gene product to be produced in a living cell substantially only when an inducer which corresponds to the promoter is present in the cell. A "tissue-specific" promoter is a nucleotide sequence which, when operably linked with a polynucleotide which encodes or specifies a gene product, causes the gene product to be produced in a living cell substantially only if the cell is a cell of the tissue type corresponding to the promoter.

The term "protein" typically refers to large polypeptides. Conventional notation is used herein to portray polypeptide sequences: the left-hand end of a polypeptide sequence is the amino-terminus; the right-hand end of a polypeptide sequence is the carboxylterminus.

A "recombinant polypeptide" is one which is produced upon expression of a recombinant polynucleotide.

The term "regulate" refers to either stimulating or inhibiting a function or activity of interest.

As used herein, term "regulatory elements" is used interchangeably with "regulatory sequences" and refers to promoters, enhancers, and other expression control elements, or any combination of such elements.

As used herein, the term "mammal" refers to any member of the class Mammalia, including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be included within the scope of this term.

The term "subject" as used herein refers to a member of species for which treatment and/or prevention of a disease or disorder using the compositions and methods of the presently disclosed subject matter might be desirable. Accordingly, the term "subject" is intended to encompass in some embodiments any member of the Kingdom Animalia including, but not limited to the phylum Chordata (e.g., members of Classes Osteichthyes (bony fish), Amphibia (amphibians), Reptilia (reptiles), Aves (birds), and Mammalia (mammals), and all Orders and Families encompassed therein. A subject is preferably a human, most preferably a human male, even more preferably an adult human male.

As used herein, the terms "vector", "cloning vector", and "expression vector" refer to a vehicle by which a polynucleotide sequence (e.g., a foreign gene) can be introduced into a host cell, so as to transduce and/or transform the host cell in order to promote expression (e.g., transcription and translation) of the introduced sequence. Vectors include plasmids, phages, viruses, etc.

All genes, gene names, and gene products disclosed herein are intended to correspond to homologs and/or orthologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates.

In **a first aspect,** the invention pertains to a compound or composition for use in the treatment of a cardiovascular disease in a subject, wherein the compound or composition is, or comprises, an agonist of expression, function and/or stability of a gene, or protein product of a gene, selected from *Ribosomal Protein S4 Y-Linked 1 (RPS4Y1), DEAD-Box Helicase 3 Y-linked (DDX3Y), Eukaryotic Translation Initiation Factor 1A Y-linked* (*EIF1AY*)*, Ubiquitously Transcribed Tetratricopeptide Repeat Containing Y-linked (UTY), Zink Finger Protein Y-linked* (*ZFY*), *Lysine Demethylase 5D (KDM5D), Ubiquitin Specific Peptidase 9 Y-linked (USP9Y)* and the non-coding genes *Testis Expressed Transcript Y-linked 14 (TTTY14)* and *Long Intergenic Non-Coding RNA 278 (LINC00278).*

In a first alternative of the first aspect, the invention also pertains to a method of treating a subject suffering from a cardiovascular disease, the method comprising a step of administering to the subject a compound or composition which comprises or is an agonist of an expression, function and/or stability of a gene, or protein product of a gene, selected from *Ribosomal Protein S4 Y-Linked 1 (RPS4Y1), DEAD-Box Helicase 3 Y-linked* (*DDX3Y*), *Eukaryotic Translation Initiation Factor 1A Y-linked* (*EIF1AY*)*, Ubiquitously Transcribed Tetratricopeptide Repeat Containing Y-linked (UTY), Zink Finger Protein Y-linked (ZFY), Lysine Demethylase 5D (KDM5D), Ubiquitin Specific Peptidase 9 Y-linked (USP9Y)* and the non-coding genes *Testis Expressed Transcript Y-linked 14 (TTTY14)* and *Long Intergenic Non-Coding RNA 278* (*LINC00278*). Preferably such compound or composition is administered in a therapeutically effective amount to the subject.

In context of the present invention the following genes and their products are relevant:

### RPS4Y1:

The gene *Ribosomal Protein S4 Y-Linked 1 (RPS4Y1)* is located at position Yp11.2 and accessible at the HUGO gene nomenclature database (www.genenames.org) under accession no. HGNC:10425. Its gene sequence can be derived from NCBI gene sequence database under gene ID no 6192, (Version of 5-Mar-2024). The gene encodes for protein product of which isoforms and amino acid sequences can be obtained from the UniProt database under accession no. P22090.

### DDX3Y:

The gene *DEAD-Box Helicase 3 Y-Linked (DDX3Y)* is located at position Yq11.221 and accessible at the HUGO gene nomenclature database (www.genenames.org) under accession no. HGNC:2699. Its gene sequence can be derived from NCBI gene sequence database under gene ID no 8653, (Version of 26-Mar-2024). The gene encodes for protein product of which isoforms and amino acid sequences can be obtained from the UniProt database under accession no. 015523.

### EIF1AY:

The gene *Eukaryotic Translation Initiation Factor 1A Y-Linked (EIF1AY)* is located at position Yq11.223 and accessible at the HUGO gene nomenclature database (www.genenames.org) under accession no. HGNC:3252. Its gene sequence can be derived from NCBI gene sequence database under gene ID no 9086, (Version of 26-Mar-2024). The gene encodes for protein product of which isoforms and amino acid sequences can be obtained from the UniProt database under accession no. 014602.

### UTY:

The gene *Ubiquitously Transcribed Tetratricopeptide Repeat Containing, Y-Linked (UTY)* is located at position Yq11.221 and accessible at the HUGO gene nomenclature database (www.genenames.org) under accession no. HGNC:12638. Its gene sequence can be derived from NCBI gene sequence database under gene ID no 7404, (Version of 26-Mar-2024). The gene encodes for protein product of which isoforms and amino acid sequences can be obtained from the UniProt database under accession no. 014607.

### ZFY:

The gene *Zinc Finger Protein Y-Linked (ZFY)* is located at position Yp11.2 and accessible at the HUGO gene nomenclature database (www.genenames.org) under accession no. HGNC:12870. Its gene sequence can be derived from NCBI gene sequence database under gene ID no 7544, (Version of 26-Mar-2024). The gene encodes for protein product of which isoforms and amino acid sequences can be obtained from the UniProt database under accession no. P08048 .

### KDM5D:

The gene *Lysine Demethylase 5D (KDM5D)* is located at position Yq11.223 and accessible at the HUGO gene nomenclature database (www.genenames.org) under accession no. HGNC:11115. Its gene sequence can be derived from NCBI gene sequence database under gene ID no 8284, (Version of 26-Mar-2024). The gene encodes for protein product of which isoforms and amino acid sequences can be obtained from the UniProt database under accession no. Q9BY66.

### USP9Y:

The gene *Ubiquitin Specific Peptidase 9 Y-Linked (USP9Y)* is located at position Yq11.221 and accessible at the HUGO gene nomenclature database (www.genenames.org) under accession no. HGNC:12633. Its gene sequence can be derived from NCBI gene sequence database under gene ID no 8287, (Version of 26-Mar-2024). The gene encodes for protein product of which isoforms and amino acid sequences can be obtained from the UniProt database under accession no. O00507.

### TTTY14:

The gene *Testis Expressed Transcript, Y-Linked 14 (TTTY14)* is located at position Yq11.222 and accessible at the HUGO gene nomenclature database (www.genenames.org) under accession no. HGNC:18495. Its gene sequence can be derived from NCBI gene sequence database under gene ID no 83869, (Version of 26-Mar-2024).

### LINC00278:

The gene *Long Intergenic Non-Protein Coding RNA 278 (LINC00278)* is located at position Yp11.31 and accessible at the HUGO gene nomenclature database (www.genenames.org) under accession no. HGNC:38712. Its gene sequence can be derived from NCBI gene sequence database under gene ID no 100873962, (Version of 26-Mar-2024).

In a preferred embodiment of the invention the compound or composition is an agonist of the expression of a protein product of the gene, preferably the agonist is protein comprising the sequence of the protein product of the gene or is a nucleic acid construct for the expression of the protein product of the gene.

RNA and amino acid sequences of the products of the above genes are provided in the sequence listing and Table 1 below. Such sequences can be used for the design of expression constructs and vectors for gene therapy or for recombinant production of agonists of the invention (IncRNA or protein).

The design of expression constructs for proteins is indeed a standardized process in molecular biology. This process involves selecting and assembling various genetic elements in a DNA construct to efficiently produce a protein of interest. The person of skill knows to adapt the technology to the specific scenario, no inventive skill is required for putting the invention into practice using the common general knowledge and the disclosure of the present invention. For example the following elements can be included in expression constructs: Promoter: A sequence of DNA needed to turn a gene on or off. Selection of a promoter depends on the desired expression level and the system in which the protein will be expressed (bacterial, yeast, insect, mammalian cells, etc.). For high-level expression, strong promoters are used, while for regulated expression, inducible promoters might be preferred. Gene of Interest: This is the DNA sequence that codes for the protein or RNA to be expressed. The gene sequence may be optimized for expression in the target host, considering factors such as codon usage, mRNA secondary structures, and GC content. Gene sequences can be obtained by databases mentioned elsewhere herein. Further provided are RNA and amino acid sequences with which the person of skill can engineer expressible sequences that encode for such products. Signal Peptide (if applicable): If the protein needs to be secreted out of the cells, a signal peptide sequence may be added to the N-terminus of the protein to direct it through the secretory pathway. In the present invention secretion signals are particularly preferred. Epitope Tags: Short peptide sequences that can be added to either the N- or C-terminus of the protein for detection or purification purposes using specific antibodies. Multiple Cloning Site (MCS): A region containing multiple unique restriction sites that allows for the easy insertion of the gene of interest. Selection Marker: Genes that confer resistance to antibiotics or other selection agents to ensure that only cells that have taken up the expression construct survive, facilitating the isolation of transfectants. Terminator Sequence: A sequence that signals the end of transcription. This is important for proper polyadenylation in eukaryotic systems and for terminating transcription to prevent run-through from the insert into other plasmid sequences. Replication Origin: Necessary for plasmid maintenance and replication within the host cells. The choice of origin depends on the host system. Optional Elements: Depending on the specific needs, other elements such as enhancers, insulators, introns, and specialized tags for purification (like His-tags) or protein-protein interaction studies (like GST-tags) may be included. The construction of expression vectors is facilitated by a variety of cloning techniques, including traditional restriction enzyme digestion and ligation, seamless cloning methods (like Gibson Assembly), and site-specific recombination systems (like Gateway cloning).

For gene therapeutic applications may use viral vectors such as engineered viruses with their disease-causing genes removed and replaced with therapeutic genes. Common types include adenoviruses, adeno-associated viruses (AAV), lentiviruses, and retroviruses. Also non-viral vectors can be employed. These include DNA plasmids, lipid nanoparticles, and other synthetic carriers designed to transport DNA or RNA into cells without using a viral backbone.

In context of the invention, the subject is preferably a male subject, preferably a human male patient, most preferably an adult human male. In certain embodiments the subject is 40 years or older, preferably is 50 years or older.

In context of the present invention it may be preferably that the subject or cells of the subject suffer from a mosaic loss of Y chromosome (mLoY) in peripheral blood cells.

Most preferred in context of the invention is that the agonist is a Ying Yang 1 Binding Motif (YY1BM) micropeptide or a derivate thereof. In particular wherein the derivative thereof is selected from a micropeptide having the same amino acid sequence, but wherein the peptide is chemically modified, for example to increase stability or bioavailability. Alternatively the derivative of the micropeptide comprises one, two or three amino acid substitutions, additions and/or deletions compared to the wild-type sequence. Another derivative is a micropeptide that is fused to a second amino acid sequence, either N or C terminally.

For example in certain embodiments of the invention, the therapy is a gene therapy and involves introducing an expression construct of one or more of the genes into a cell of the subject.

In another embodiment of the invention the treatment involves administration of a genetically modified cell to the subject, wherein the genetically modified cell is modified to express one or more of the genes.

A cell is preferably a hematopoietic cell such as a macrophage.

In **a second aspect,** the invention pertains to a method for identifying a modulator cell-mediated regulation of myocardial cell function, the method comprising the steps:
- Providing a first cell and a second cell, wherein the second cell is a myocardial cell;
- Culturing the first cell to obtain a test first cell under conditions which are selected from:
   (i) where the expression one or more known candidate genes are being perturbed, preferably knocked out or knocked in, in the first cell:
   (ii) With one or more genetic expression constructs being expressed in the first cell to overexpress, or ectopically express, one or more known candidate genes in the first cell; and
   (iii) Where the first cell is cultured in contact with one or more candidate compounds
- optionally culturing a control first cell under control conditions (not the conditions of step (b));
- Bringing into contact the second cell and the test first cell, or the second cell and conditioned medium of the culture of the test first cell, and, optionally, culturing the second cell under the contact conditions:
Wherein a difference of the test first cell to a control or control first cell in one or more markers of myocardial function indicates that the one or more known candidate genes or compounds is a modulator of a cell mediated regulation of myocardial cell function.

Perturbation of a candidate gene may be realized to any genetic method known in the field and may include in particular ectopic expression of test gene sequences, ectopic expression of dominant negative sequences of test genes, expression of genetic constructs for the targeted knock-down or knock out of genes, for example using RNA interference or gene edition. Public libraries today provide predesigned knock out and knock down complementary RNA sequences (for siRNA, shRNA, guideRNA etc). Hence, it is within the ambit of the person of skill to design and express such perturbation constructs in the screening system of the present invention.

Preferably, the marker of myocardial function is selected from beating frequencies of cardiomyocytes, established protein markers of fibroblast activation or expression of known cell-cell interaction protein.

The first cell is preferably selected from an immune cell such as a PBMC, a PBMC-derived or THP-1 monocyte, in particular the first cell is a macrophage such as an M0 macrophage.

The second cell is preferably selected from a primary human cardiac fibroblasts (HCF), neonatal rat cardiomyocytes (NRCM) and human umbilical vein endothelial cells (HUVEC).

In preferred embodiments of the second aspect the known candidate gene is a (human) Y-chromosome located gene.

In **a third aspect,** the invention pertains to a method for the identification of a compound useful in the treatment or prevention of a cardiovascular disorder, the method comprising the step of:
- Providing a candidate compound, composition, or a candidate medical procedure;
- Providing a test cell or test tissue, wherein the test cell or test tissue is involved with or is associated with the cardiovascular disease;
- Contacting the test cell or test tissue with the candidate compound, composition or performing on the test cell or test tissue the candidate medical procedure;
- Determining in the test cell or test tissue a test gene expression level, or a level of protein, wherein the protein is expressed by such test gene, the test gene being selected from the group consisting of *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDM5D), Ubiquitin Specific Peptidase 9 Y-linked (USP9Y) and the non-coding genes Testis Expressed Transcript Y-linked 14 (TTTY14)* and *Long Intergenic Non-Coding RNA 278 (LINC00278);*
Wherein an increased gene expression level, or an increased protein level in the test cell or test tissue compared to a reference, or compared to a control cell or control tissue not contacted with the candidate compound or composition or medical procedure, indicates that the respective candidate compound or composition or candidate medical procedure is useful in the treatment or prevention of a cardiovascular disease.

As used herein, the term "candidate compound", "candidate substance", "test compound" or "test agent" refers to any compound, molecule or agent that is to be tested. As used herein, the terms, which are used interchangeably, refer to biological or chemical compounds such as simple or complex organic or inorganic molecules, small molecules, peptides, proteins, oligonucleotides, polynucleotides, carbohydrates, or lipoproteins. A vast array of compounds can be synthesized, for example oligomers, such as oligopeptides and oligonucleotides, and synthetic organic compounds based on various core structures, and these are also included in the terms noted above. In addition, various natural sources can provide compounds for screening, such as plant or animal extracts, and the like. Compounds can be tested singly or in combination with one another. Agents or candidate compounds can be randomly selected or rationally selected or designed. As used herein, an agent or candidate compound is said to be "randomly selected" when the agent is chosen randomly without considering the specific interaction between the agent and the target compound or site. As used herein, an agent is said to be "rationally selected or designed", when the agent is chosen on a nonrandom basis which takes into account the specific interaction between the agent and the target site and/or the conformation in connection with the agent's action. In some embodiments, the assays described herein can be used to guide a rational design, for example, by providing mechanistic insight into the efficacy of a test compound which is feed in an iterative fashion into a series of assays or screens while refining the selection of test compounds. A test compound can be a control compound.

As used herein, the term "small molecule" can refer to compounds that are "natural product-like," however, the term "small molecule" is not limited to "natural product-like" compounds. Rather, a small molecule is typically characterized in that it contains several carbon- carbon bonds, and has a molecular weight more than about 50, but less than about 5000 Daltons (5 kD). Preferably the small molecule has a molecular weight of less than 3 kD, still more preferably less than 2 kD, and most preferably less than 1 kD. In some cases, it is preferred that a small molecule have a molecular mass equal to or less than 700 Daltons.

In some preferred embodiments the candidate compound or composition comprises a nucleic acid construct for the expression of protein or peptide sequence, wherein the protein or peptide sequence comprises an amino acid sequence, or a fragment thereof, encoded by any one of the test genes. In particular such genetic sequence of a test gene is derived or identical to the respective sequences provided herein in the sequence listing.

In some embodiments the candidate compound or composition comprises a protein or peptide, wherein the protein or peptide comprises an amino acid sequence, or a fragment thereof, encoded by any one of the test genes. Also protein sequences of the test genes are provided herein in the attached sequence listing.

In **a fourth aspect,** the invention pertains to a method for diagnosing a subject, the method comprising determining in a biological sample of the subject the level of gene or protein expression of at least one biomarker selected from *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDM5D), Ubiquitin Specific Peptidase 9 Y-linked (USP9Y) and the non-coding genes Testis Expressed Transcript Y-linked 14 (TTTY14)* and *Long Intergenic Non-Coding RNA 278 (LINC00278)* or its encoded micropeptide YY1 BM; wherein a reduced gene or protein expression indicates an increased risk of cardiovascular disease in the subject.

In **a fifth aspect,** the invention pertains to a use of a biomarker for the diagnosis of an increased risk of cardiovascular disease in a subject, wherein the protein expression or gene expression is selected from an expression of the genes *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDMSD), Ubiquitin Specific Peptidase 9 Y-linked (USP9Y) and the non-coding genes Testis Expressed Transcript Y-linked 14 (TTTY14)* or *Long Intergenic Non-Coding RNA 278 (LINC00278).*

In preferred embodiments of all aspects of the present invention a The method of any one of the preceding claims, wherein the gene or test gene is selected from *UTY, ZFY, TTTY14* and *LINC00278,* and preferably is *LINC00278.*

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

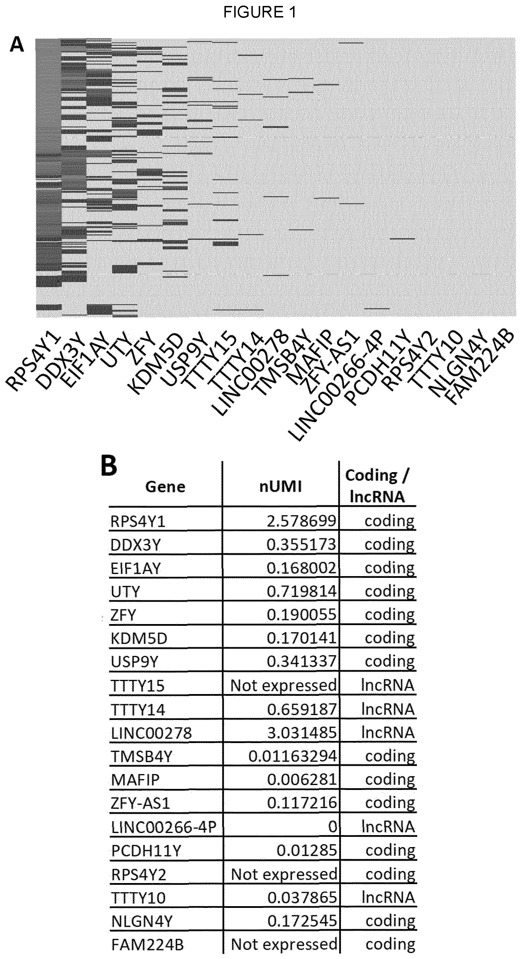

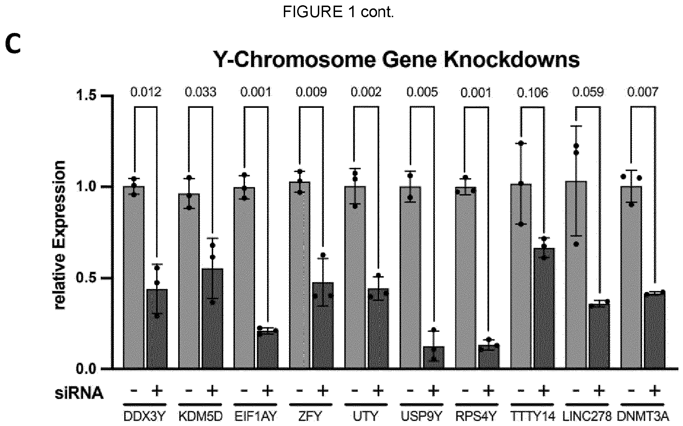

**Figure 1****: Y-Chromosomal Gene Expression Patterns are consistent across different models. A** Single Cell RNA Sequencing (scRNA-Seq; 10X Genomics Chromium platform) analyses from PBMCs obtained from male patients undergoing transcatheter aortic valve replacement for severe aortic valve replacement. Depicted are nUMI of the top expressed Y-chromosomal genes. **B** THP-1 M0 macrophages were subjected to scRNA-Seq, the nUMI of the indicated genes are shown. **C** THP-1 M0 macrophages were transfected with pools of four siRNAs targeting the indicated genes. Knockdown efficacy was confirmed by RT-qPCR using primer pairs for the indicated genes. Numbers indicate p values as determined by Welch's test.

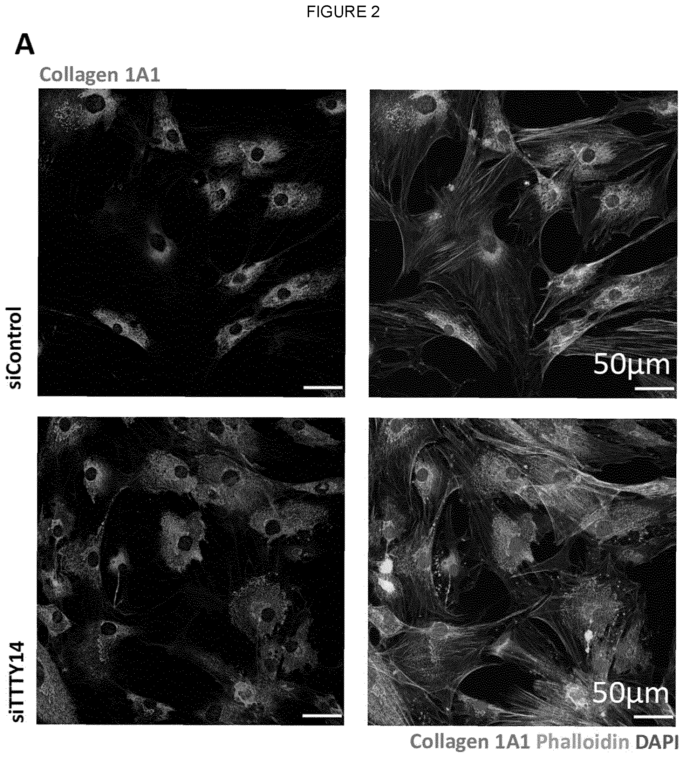

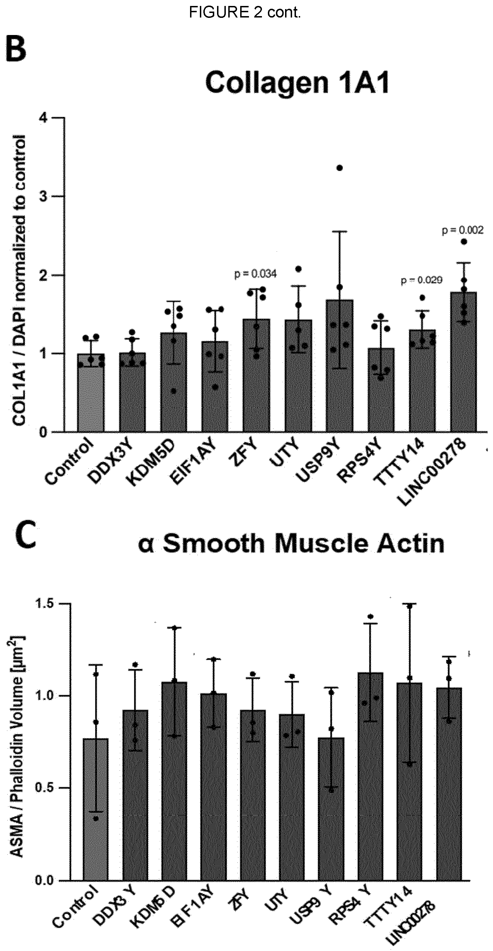

**Figure 2****: Loss of Y-chromosomal genes induces late paracrine activation of primary human cardiac fibroblasts.** The indicated genes were silenced in THP-1 M0 macrophages and conditioned media collected. Primary human cardiac fibroblasts were treated with the respective conditioned medium for 72 hours. **A:** Representative IHC images with staining for DAPI, Phalloidin and Collagen 1A1 of control siRNA and siRNA targeting *TTTY14.* **B, C:** Quantification of Collagen 1A1 and α Smooth Muscle Actin were performed. Indicated p values determined by Kruskal-Wallis-Test and Dunn's post-hoc test.

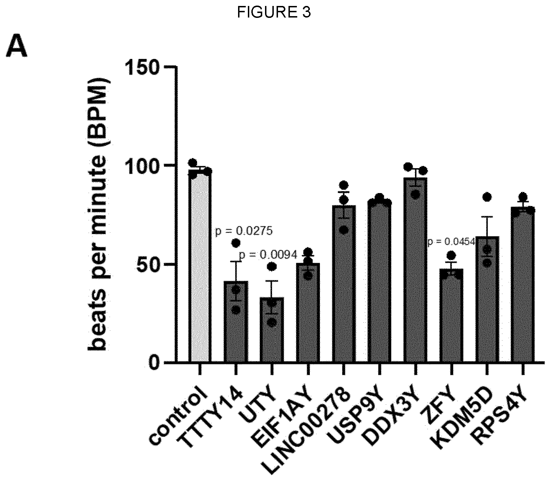

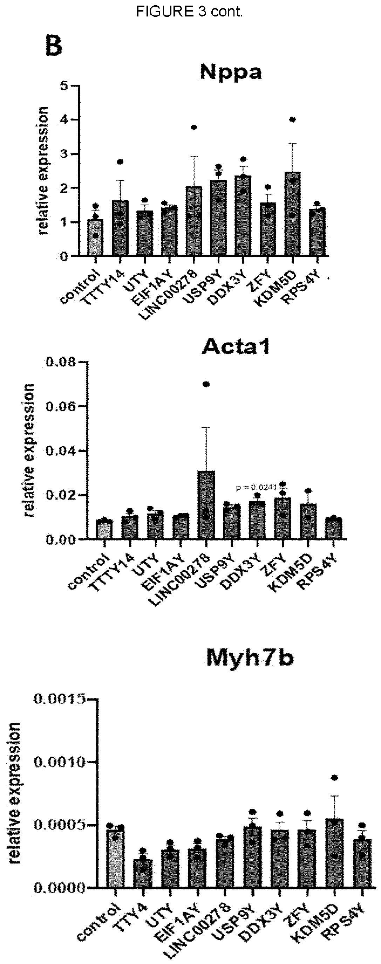

**Figure 3****: Loss of Y-chromosomal genes in macrophages induces alterations in cardiomyocyte beating frequencies in a paracrine manner.** siRNAs were transfected into THP-1 M0 macrophages to silence the indicated genes. Conditioned media were collected and used to treat neonatal rat cardiomyocytes (NRCM). **A:** Beating frequencies of NRCMs were measured. **B:** mRNA contents of the indicated genes were measured in NRCMs. Indicated p values were calculated by Kruskal-Wallis test and Dunn's post-hoc test.

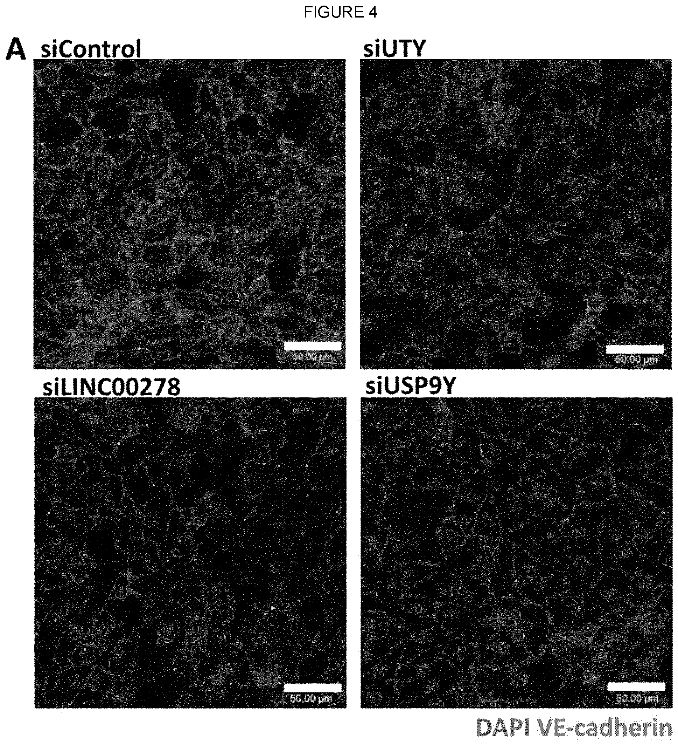

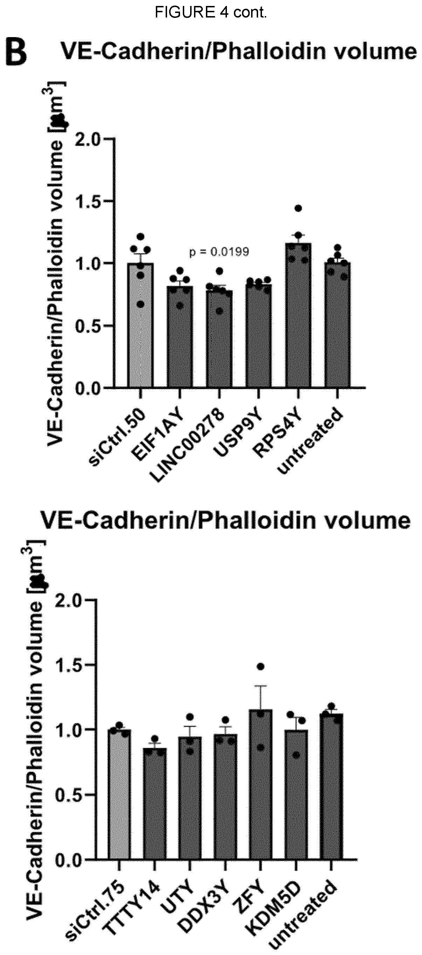

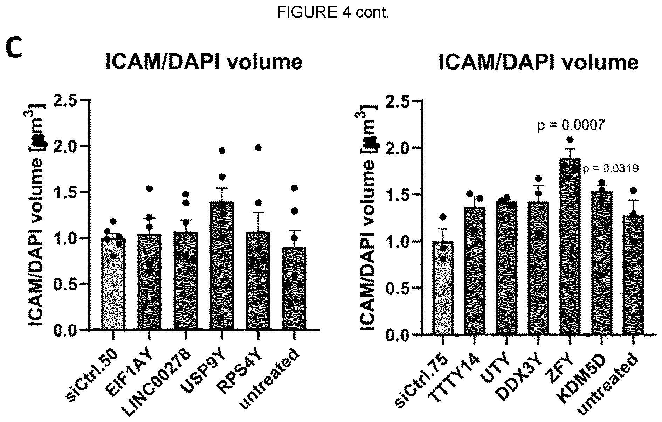

**Figure 4****: Loss of Y-chromosomal genes in macrophages induces endothelial activation and impaired junction integrity.** The indicated genes were silenced in THP-1 M0 macrophages using siRNA pools. Conditioned media were collected and used to treat human umbilical vein endothelial cells (HUVECs) for 48 hours. **A:** Representative images of HUVECs treated with conditioned media from macrophages transfected with the indicated siRNA pools and stained for DAPI and VE-cadherin. **B:** Image-based quantification of VE-cadherin expression in HUVECs treated with conditioned media from THP-1 M0 macrophages with the indicated genes silenced. Treatment groups were normalized to the respective concentration of control siRNA in the gene silencing in THP-1 M0 macrophages. C: Quantification of ICAM expression in HUVECs after treatment with conditioned media from THP-1 macrophages silenced for the indicated genes. Expression levels were normalized to the respective control siRNAs using the according concentrations in gene silencing in THP-1 M0 macrophages.

The sequences show:

**Table 1: Sequences of the Invention**

| **SEQ ID NO:** | **Gene Name** | **Protein or RNA** | **Description** |
|---|---|---|---|
| 1 | ***RPS4Y1*** | PROT | Small ribosomal subunit protein eS4, Y isoform 1 |
| 2 | ***RPS4Y1*** | RNA | Human ribosomal protein (RPS4Y) isoform mRNA, complete cds |
| 3 | ***DDX3Y*** | PROT | ATP-dependent RNA helicase DDX3Y OS=Homo sapiens |
| 4 | ***DDX3Y*** | RNA | Homo sapiens dead box, Y isoform (DBY) mRNA, alternative transcript 1, complete cds. |
| 5 | ***DDX3Y*** | RNA | Homo sapiens dead box, Y isoform (DBY) mRNA, alternative transcript 2, complete cds. |
| 6 | ***EIFIAY*** | PROT | Eukaryotic translation initiation factor 1A, Y-chromosomal OS=Homo sapiens |
| 7 | ***EIFIAY*** | RNA | Homo sapiens eIF-1A, Y isoform (EIF1AY) mRNA, complete cds. |
| 8 | ***UTY*** | PROT | UTY_HUMAN Histone demethylase UTY OS=Homo sapiens |
| 9 | ***UTY*** | RNA | Homo sapiens ubiquitous TPR motif, Y isoform (UTY) mRNA, alternative transcript 1, complete cds. |
| 10 | ***ZFY*** | PROT | ZFY_HUMAN Zinc finger Y-chromosomal protein OS=Homo sapiens |
| 11 | ***ZFY*** | RNA | Human zinc finger protein Y-linked (ZFY) mRNA, complete cds. |
| 12 | ***KDM5D*** | PROT | Lysine-specific demethylase 5D OS=Homo sapiens |
| 13 | ***KDM5D*** | RNA | Human SMCY (H-Y) mRNA, complete cds. |
| 14 | ***USP9Y*** | PROT | Probable ubiquitin carboxyl-terminal hydrolase FAF-Y |
| 15 | ***USP9Y*** | RNA | Homo sapiens chromosome Y ubiquitin specific protease 9 (USP9Y) mRNA, complete cds |
| 16 | ***TTTY14*** | RNA | Homo sapiens testis expressed transcript, Y-linked 14 (TTTY14), transcript variant 1, long non-coding RNA |
| 17 | ***TTTY14*** | RNA | Homo sapiens testis expressed transcript, Y-linked 14 (TTTY14), transcript variant 2, long non-coding RNA |
| 18 | ***TTTY14*** | RNA | Homo sapiens testis expressed transcript, Y-linked 14 (TTTY14), transcript variant 3, long non-coding RNA |
| 19 | ***TTTY14*** | RNA | Homo sapiens testis expressed transcript, Y-linked 14 (TTTY14), transcript variant 4, long non-coding RNA |
| 20 | ***TTTY14*** | RNA | Homo sapiens testis expressed transcript, Y-linked 14 (TTTY14), transcript variant 5, long non-coding RNA |
| 21 | ***TTTY14*** | RNA | Homo sapiens testis expressed transcript, Y-linked 14 (TTTY14), transcript variant 6, long non-coding RNA |
| 22 | ***TTTY14*** | RNA | Homo sapiens testis expressed transcript, Y-linked 14 (TTTY14), transcript variant 7, long non-coding RNA |
| 23 | ***TTTY14*** | RNA | Homo sapiens testis expressed transcript, Y-linked 14 (TTTY14), transcript variant 8, long non-coding RNA |
| 24 | ***LINC00278*** | RNA | Homo sapiens long intergenic non-protein coding RNA 278 (LINC00278), long non-coding RNA |

### EXAM PLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Identification of Y-chromosomal genes using a paracrine screening assay

To identify Y-chromosomal genes, whose loss induces juxta- or paracrine effects on myocardial cell populations, the inventors developed a screening workflow. In a first step, single cell RNA sequencing (scRNA-Seq) data from both human PBMC-derived monocytes and the monocytic THP-1 cell line were screened for the consistently highest expressed Y-chromosomal genes. PBMCs were obtained from patients undergoing transcatheter aortic valve replacement for severe aortic valve stenosis. Using the 10X Genomics Chromium platform, scRNA-Seq allows to investigate transcriptomes of each cell in a sample individually. The nine genes with the consistently highest expression between the two models were *Ribosomal Protein S4 Y-Linked 1 (RPS4Y1), DEAD-Box Helicase 3 Y-linked (DDX3Y), Eukaryotic Translation Initiation Factor 1A Y-linked* (*EIF1AY*)*, Ubiquitously Transcribed Tetratricopeptide Repeat Containg Y-linked* (*UTY*), *Zink Finger Protein Y-linked* (*ZFY*), *Lysine Demethylase 5D* (*KDM5D*), *Ubiquitin Specific Peptidase 9 Y-linked (USP9Y)* and the non-coding genes *Testis Expressed Transcript Y-linked 14 (TTTY14)* and *Long Intergenic Non-Coding RNA 278 (LINC00278)* (Fig. 1 A, B).

### Example 1: Identification of Y-chromosomal genes using a paracrine screening assay

Using pools of four small interfering RNAs (siRNAs) targeting each gene, the inventors silenced the identified genes individually in THP1 M0 macrophages with sufficient silencing efficacy (Fig. 1C). Conditioned medium (CM) from these macrophages was collected and used to treat primary human cardiac fibroblasts (HCF), neonatal rat cardiomyocytes (NRCM) and human umbilical vein endothelial cells (HUVEC).

In THP1 M0 macrophages, no changes in mRNA content of the pro-fibrotic marker genes *TGFB1* and *TGFB2* or the pro-inflammatory marker genes *IL6, TNF* or *IL1B* was observed. However, the inventors found a robust induction of collagen protein expression in HCF treated with CM from macrophages silenced for *ZFY, TTTY14* and *LINC00278* for 72 hours (Fig. 2A, B). However, upon CM treatment for 72 hours, changes in α smooth muscle actin protein expression (Fig. 2C) were not observed.

Treatment with CM from *TTTY14-, UTY-* and *ZFY*-silenced macrophages for 24 hours resulted in lower beating frequencies in NRCM while not affecting cell size or mRNA expression of cardiomyocyte stress-marker genes (*Nppa, Acta1, Myh7b*) (Fig. 3). Treating HUVEC with CM from macrophages silenced for *LINC00278* resulted in lower protein content of VE-Cadherin, a classical cell-cell adhesion glycoprotein, hence indicative of impaired cell-junction integrity. (Fig. 4A, B). Treatment with CM from THP-1 M0 macrophages silenced for *ZFY* and *KDM5D* induced increased protein expression of Intercellular Adhesion Molecule (ICAM), a canonical endothelial cell activation marker (Fig. 4C).

These data indicate that different Y-chromosome encoded genes in monocytic cells exert protective effects on different mesenchymal cardiac cell types in a juxta- or paracrine manner. Particularly *UTY, ZFY, TTTY14* and *LINC00278* appear to play a major role in mediating effects of hematopoietic cells.

These findings may lead the way towards a better understanding of mechanisms underlying mLoY and therapy development for affected patients. Overexpression e.g. by modified RNAs or interfering with down-stream target of these genes may improve cardiovascular disease progression in patients with LoY. Especially *LINC00278,* which is a human specific long non-coding RNA, encodes a micropeptide [13], which could be delivered systemically and may provide cardioprotective effects.

### Example 2 [prophetic]: Rescue in Y-chromosome deficient induced pluripotent stem cells (iPSCs)

The inventors use induced pluripotent stem cell (iPSC) techniques. Using the CRISPR/Cas9 system, the Y chromosome will be deleted from human iPSCs, which will subsequently be differentiated into macrophages and subsequently incubated with lipid nanoparticles containing mRNAs encoding *LINC00278, ZFY* or *TTTY14.* Delivery of the respective genes will be assessed via RT-qPCR. The sequences are shown below. Following cultivation over a period of 4 days, collected supernatants will be used to treat myocardial cell populations. Primary human cardiac fibroblasts will be assessed for activation markers (alpha smooth muscle actin and collagen 1A1) using immunohistochemistry. iPSC-derived cardiomyocytes will be investigated for stress markers, especially cellular hypertrophy, beating frequency and mRNA expression of stress markers, i.e. MYH7B, ACTA1, NPPA, NPPB. Additionally, human umbilical vein endothelial cells (HUVEC) will be assessed for cell junction integrity and activation, using IHC for VE-Cadherin, ICAM-1 and VCAM.

Using an iPSC-derived self-organised human cardiac organoid system containing cardiomyocytes, endothelial cells and fibroblasts, the inventors will investigate the effects of LoY macrophages on a more systemic level. LoY iPSC-derived macrophages and according controls will be co-cultured with cardiac organoids for 7 days. Upon start of co-culture, the three hematopoietic cell-targeting lipid nanoparticle-mRNAs as indicated before will be added..

Following co-culture, beating frequencies, organoid hypertrophy - indicative for cardiomyocyte hypertrophy - as well as fibroblast and endothelial cell activation will be assessed as described before. Additionally, the infiltration of macrophages into the organoids will be investigated via IHC for CD68.

Sequences used in this example:
*LINC00278* sequence (5'-3') shown as RNA: (SEQ ID NO: 25) 
UGGUGAGCCAGCCAGGAGUGAAGACGACAGGUUUGCUGUCUCCUUUGCCUGUG
GGUCUGGGGCCCCAGCCUGGGGGAGACCCGUGACUCCAGGCAUGCUAUCAGGACAACU
UCAACCCGAGGGGAGAUCAGCUCUCCCGCAACCUGGUGCCGCUCUCUGACAGCACAAC
AGAACCUAAGGGGCUACAGGAUGAUUCAAGGAACAGUGUGCUACAGGACCUCGCAAGAA
GACCCUCACCAGAUGCAGCCCCUCUAUGUUGGACUUCCCAGCCUCCGGAAGUUAGAAA
UGUGCUACAACCACUCAAGGUCUGCAAACAGAUUCCUAUUUAGAUGUUGUUGAGAGCUG
AAUUUCUGGAACAAGAGAUAUAGAUUAAGACUGUUGUUACAUGUUGCCUACAUGACUUU
CAAAAGCUUGAUUCCCAGUGGCGCUCUCAACAGCAGUCAUAAACAAUGGUAAUUCAAUG
UAUAUUUAUAUUCAACUGAUAUAUUAAUUGGAAUAUAUUAAAAAUACGUAUAUUUAUAUU
CAGAAUAAAAAAAA
*TTTY14* sequence (5'-3') shown as RNA: (SEQ ID NO: 26) 
AAUCGUCAAUGCGACGUCCUCCUACUCCCACCUCAGAGCUGUCACAGAGACUGU
CAGUUCCGUCCCUCCCCUUCAGCCCUUUAACGCACGUCUUCCCGACCGCAUAACUUGC
UCAGUCCCUGCGGCCAACUGGGGCGGGCCAAUGGCUGUGACGGAUAAGGGGUGUGGG
GUGCGGGGACAAUGGGCCAGAGCUUCUCAUGCAGCCUGCACCAGCGCCGGGUCGGAG
AGUCAGAGGCCACCCUGAGAUGGACCGAGAUCUUCAGUUGGGGAGGCGCCUCCGGUGA
GCUUGCGGCCAGAUGCCCCCUCAGUAGUCCUCCGCAACAGGUGCCGCGCAGCCGAGUG
GCGGGCUAGCAUACCGCCUGCCCGCAAUUCGGCCAGGCCUCGAGCCUGCUCCGCCUUC
CCGCUGCGGUAGCGCCGCCGGGGAAACGCCGCCACGGGCUGCAUGGGCAGCGCUCGC
GCCCCGCCGCUGAGCCGCCGGGGAGCCGUCCAGUCUUCGAAUCCCAGCUUGGUGGCA
UGCAUGUUUAGUCCCAGCUACACAGCAGGGAGGCUGAGGCUGAAGGAAUACUUGAGCC
CAGGAAUUCAAGACUAGCCUGCACAACAUAGUGAGACCCUGACUUUAAAAUAAAUUUAAA
AAAAUUAAAAGCAACAAGACUUUCUGCUUGUAUAUGAUGAUGUUAGUAUGCUGACUUAC
AAGGUUAGAUCAUUUUGAUAUGUGUGAUGUAGGUUUUCAAAGCUGGCACUGUUUUAUG
CUUCAAAUAUUAUUUUUAUUCAUUGGUUCUCAUGGGAUUUCAUCACUAAGCUGUAAAUC
AAUAAUCAUAAUGUUGAGGGGAGGGGAUUACAAAAUAGCUCUUCAAAAUCAUCUUUGCA
UUUCUCAAUAAACAGCAUUCCCUUUUGAAAAAA
*ZFY sequence (5'-3')* shown as RNA: (SEQ ID NO: 27)

AUGGAUGGUGAUCAGAUUGUUGUGGAAAUACAAGAAGCAGUUUUUGUUUCUAAU
AUUGUGGAUUCUGACAUAACUGUGCAUAACUUUGUUCCUGAUGACCCAGACUCAGUUG
UAAUCCAAGAUGUUGUUGAAGAUGUUGUCAUAGAGGAGGAUGUUCAGUGCUCAGAUAU
CUUAGAAGAGGCAGAUGUAUCUGAAAAUGUCAUCAUUCCUGAGCAAGUGCUGGACUCAG
AUGUAACUGAAGAAGUUUCUUUACCACACUGCACAGUCCCAGAUGAUGUUUUAGCUUCU
GACAUUACUUCAACCUCAAUGUCUAUGCCAGAACAUGUUUUAACGAGUGAAUCCAUGCA
UGUGUGUGACAUUGGACAUGUUGAACAUAUGGUGCAUGAUAGUGUAGUGGAAGCAGAA
AUCAUUACUGAUCCUCUGACGAGUGACAUAGUUUCAGAAGAAGUAUUGGUAGCAGACUG

UGCCCCUGAAGCAGUCAUAGAUGCCAGCGGGAUCUCAGUGGACCAGCAAGAUAAUGAC
AAAGCCAGCUGUGAGGACUACCUAAUGAUUUCGUUGGAUGAUGCUGGCAAAAUAGAACA
UGAUGGUUCCACUGGAGUGACCAUCGAUGCAGAAUCAGAAAUGGAUCCUUGUAAAGUG
GAUAGCACUUGUCCUGAAGUCAUCAAGGUGUACAUUUUUAAAGCUGACCCUGGAGAAGA
UGACUUAGGUGGAACUGUAGACAUUGUGGAGAGUGAACCUGAAAAUGAUCAUGGAGUU
GAACUACUUGAUCAGAACAGCAGUAUUCGUGUUCCCAGGGAAAAGAUGGUUUAUAUGAC
UGUCAAUGACUCUCAACAAGAAGAUGAAGAUUUAAGUAAUAAUUCUGAUGGAAUUGAAA
ACCGGAAUGGCACUGCAAGUGCCCUCUUGCACAUAGAUGAGUCUGCUGGCCUUGGCAG
ACUGGCUAAACAGAAACCAAAGAAAAAGAGAAGACCUGAUUCCAGGCAGUACCAAACAGC
AAUAAUUAUUGGCCCUGAUGGUCAUCCUUUGACUGUCUAUCCUUGCAUGAUUUGUGGG
AAGAAGUUUAAGUCGAGGGGUUUUUUGAAAAGACACAUGAAAAACCAUCCUGAACACCU
UGCCAAGAAGAAGUACCACUGUACUGACUGUGAUUACACUACCAAUAAGAAGAUAAGUU
UACAUAACCACCUGGAGAGCCACAAGCUGACCAGCAAGGCAGAGAAGGCCAUUGAAUGU
GAUGAGUGUGGGAAGCAUUUUUCUCAUGCAGGGGCUUUGUUUACUCACAAAAUGGUGC
AUAAGGAAAAAGGGGCCAACAAAAUGCACAAGUGUAAAUUCUGUGAAUAUGAGACAGCU
GAACAGGGGUUAUUGAAUCGCCACCUCUUGGCAGUCCACAGCAAGAACUUUCCUCAUAU
UUGUGUGGAGUGUGGUAAAGGUUUCCGACACCCGUCGGAACUGAGAAAGCACAUGCGA
AUCCAUACCGGCGAGAAGCCAUACCAAUGCCAGUACUGUGAAUAUAGGUCUGCAGACUC
UUCUAACUUGAAAACACAUAUAAAAACAAAGCAUAGUAAAGAGAUGCCAUUCAAGUGUGA
CAUUUGUCUUCUGACUUUCUCAGAUACCAAAGAAGUGCAGCAACAUACUCUUGUCCACC
AAGAAAGCAAAACACAUCAGUGUUUGCAUUGCGACCACAAGAGUUCAAACUCAAGUGAU
UUGAAACGACAUGUAAUUUCAGUUCAUACGAAAGACUAUCCUCAUAAGUGUGAGAUGUG
CGAGAAAGGCUUUCACAGGCCUUCAGAACUUAAGAAACAUGUGGCUGUCCACAAAGGUA
AAAAAAUGCACCAAUGUAGACAUUGUGACUUUAAGAUUGCAGACCCAUUUGUUCUAAGU
CGCCAUAUUCUCUCAGUUCACACAAAGGAUCUUCCAUUUAGGUGUAAGAGAUGUAGAAA
GGGAUUUAGGCAACAAAAUGAGCUUAAAAAGCAUAUGAAGACACACAGUGGCAGGAAAG
UAUAUCAGUGUGAGUACUGUGAGUAUAGCACUACAGAUGCCUCAGGCUUUAAACGGCAC
GUUAUUUCCAUUCAUACAAAAGACUAUCCUCAUCGGUGUGAGUACUGCAAGAAAGGCUU
CCGAAGACCUUCAGAAAAGAACCAGCACAUAAUGAGACACCAUAAAGAAGUUGGUCUGC
CCUAA

### Materials and Methods

### Single cell RNA sequencing datasets

A single cell RNA sequencing (scRNA-Seq) dataset was obtained from patient-derived peripheral blood mononuclear cells (PBMCs), facilitated by Professor WT Abplanalp. PBMCs were isolated via density gradient centrifugation by layering blood samples onto Pancoll human solution (PAN-Biotech) and centrifuging at 800g for 20 minutes at room temperature. Thrombocytes were removed through low-speed centrifugation post PBMC-extraction, followed by three washing steps in preparation for library preparation.

For scRNA-Seq of THP-1 M0 macrophages, cells were purchased from the German Collection of Microorganisms and Cell Cultures (#ACC16, DSMZ), maintained in RPMI 1640 medium (Thermo Fisher Scientific) supplemented with 10% heat inactivated fetal calf serum (Thermo Fisher Scientific), 10 mM HEPES (Sigma Aldrich) and 50 U/ml Penicillin/Streptomycin (Thermo Fisher Scientific) in a humidified incubator at 37°C with 5% CO₂. Macrophage M0 differentiation was induced by the addition of 0.1 µg/ml phorbol 12-myristate 13-acetate (PMA) for 48 hours, followed by medium exchange without PMA for an additional 24 hours. Cells were harvested using TrypLE Express (Thermo Fisher Scientific) for library preparation.

The obtained cells were used for droplet scRNA-Seq library preparation using the 10X Genomics Chromium platform and the Chromium Controller. For library preparation, the Next GEM Single Cell 3' GEM, Library and Gel Bead Kit 3.1 reagents were used (10X Genomics). Sequencing was conducted by GenomeScan using the NovaSeq 6000 platform (Illumina).

### Data analysis

Sequencing data were aligned to the human reference genome GRCh38 using the Cell Ranger suite v3 (10X Genomics). Subsequent analyses were performed utilising the Seurat package (Satija Lab) for R (v4.3.0) in R Studio Server (v2023.6.0.421). Following quality control and removal of low quality sequencing cells, unique molecular identifier counts were obtained for each Y chromosome gene and visualized in a heatmap using the ComplexHeatmap package (Zuguang Gu).

### THP-1 cell culture and macrophage differentiation

THP-1 M0 macrophages were cultured as aforementioned. Experiments were conducted in passages 4 or 5, with 5 x 10⁵ cells seeded into 6-well plates for macrophage M0 differentiation induction by supplementing the medium with 0.1 µg/ml PMA for 48 hours. After medium exchange and a subsequent 24-hour period without PMA, cells were subjected to further treatment.

### siRNA Transfection and collection of conditioned media

siRNA pools targeting four different regions of each gene were purchased from Horizon Discovery and transfected into THP-1 M0 macrophages at a concentration of 50 nM using the siTran 2.0 transfection agent (OriGene Technologies) as per the manufacturer's instructions. Medium was exchanged after 24 hours, followed by another 24-hour incubation period. Subsequently, conditioned media (CM) were collected and stored at -80°C for further use.

### Human cardiac fibroblast cell culture

Primary human cardiac fibroblasts were purchased from PromoCell and maintained in Fibroblast Growth Medium 3 (PromoCell) under standard conditions of 37°C with 5% CO₂ in a humidified incubator. For experiments, 2000 cells were seeded into 18-well µ-Slide ibiTreat chambered coverslips (ibidi) and allowed to adhere for 24 hours. Subsequently, the medium was replaced with a 1:1 mixture of the respective CM and fresh fibroblast growth medium. CM treatment durations of 48 or 72 hours were employed as indicated, followed by immunofluorescence staining.

### Isolation and Cultivation of Neonatal Rat Cardiomyocytes

Pregnant Sprague Dawley rats were obtained from Janvier Labs and maintained at a 12-hour light-dark cycle with ad libitum access to chow and water. Pups aged 1 to 3 days post-partum were sacrificed, and hearts isolated. Following transfer to Hank's buffered saline solution, hearts were minced and enzymatically dissociated using the neonatal heart dissociation kit (Miltenyi Biotec) as per the manufacturer's instructions using a gentleMACS Dissociator (Miltenyi Biotec). Cardiomyocytes and fibroblasts were pre-plated in a mixture of DMEM high glucose (BioConcept) and M199 EBS (BioConcept) supplemented with 10% horse serum (Thermo Fisher Scientific), 5% fetal calf serum (Thermo Fisher Scientific), 2% L-glutamine (Thermo Fisher Scientific) and 50 U/ml Penicillin/Streptomycin (Thermo Fisher Scientific). Cells were incubated in a humidified incubator at 37°C with 5% CO2 for 100 minutes to allow for fibroblast adhesion. The cardiomyocyte-containing supernatants were pelleted and resuspended in maintenance medium (DMEM high glucose (BioConcept) and M199 EBS (BioConcept) supplemented with 1% horse serum, 2% L-glutamine and 50 U/ml Penicillin / Streptomycin), followed by plating in 0.3 mg/ml collagen pre-coated 12-well plates at 5000 cells per well. Cells were allowed to attach for 24 hours before treatment with maintenance medium supplemented with CM in a 1:1 ratio. Life-cell imaging was performed after 24 hours of CM treatment using a Nikon Eclipse TS100 microscope, and cells were lysed for RNA isolation after 48 hours.

### Cultivation of Human Umbilical Vein Endothelial Cells (HUVEC)

HUVEC were purchased from Promocell and maintained in Endothelial Cell Basal Medium (EBM; Lonza) supplemented with EGM-SingleQuots (Lonza) and 10% fetal calf serum (Thermo Fisher Scientific) in a humidified incubator at 37°C with 5% CO₂. Experiments were conducted using cells from passages 2 or 3. 5000 cells were seeded in each well of 18-well ibiTreat µ-Slide chambered coverslips and allowed to adhere for 24 hours before treatment with CM and supplemented EBM in a 1:1 ratio for 48 hours. Subsequently, HUVEC were subjected to immunofluorescence staining.

### RNA isolation and cDNA synthesis

Cells were lysed using Qiazol reagent (Qiagen), followed by Phenol/Chloroform RNA extraction using the RNeasy Mini Kit (Qiagen) according to the manufacturer's instructions. RNA concentrations were measured using a NanoDrop One spectrophotometer (Thermo Fisher Scientific). cDNA synthesis was performed using MLV reverse transcriptase (Thermo Fisher Scientific), random hexamer primers (Thermo Fisher Scientific) and Ribolock RNase inhibitor (Thermo Fisher Scientific). The thermocycler protocol included the following steps: 65°C for 5 minutes, 25°C for 10 minutes, 37°C for 50 minutes and 70°C for 15 minutes.

### Real-time quantitative PCR

Silencing efficacy and NRCM stress response were evaluated by quantitative real time PCR (RT-qPCR) using self-designed primer pairs (sequences provided in table 2) and the Fast SYBR Green Mastermix (Applied Biosystems) in a Viia7 thermocycler (Applied Biosystems). The thermocycler protocol was as follows: 95°C for 30 seconds, 40 cycles of 95°C for 1 second, 60°C for 20 seconds. Expression data was calculated using the ΔCT method.

### Immunofluorescence staining and imaging

Cell media were removed from the chambered coverslips and cells washed once with PBS. Fixation was performed with 4% paraformaldehyde in PBS for 10 minutes. Cells were washed twice with PBS and permeabilized with 0.1% Triton X-100 (Thermo Fisher Scientific) for 10 minutes. Cells were blocked with 5% donkey serum (abcam) in PBS for 60 minutes at room temperature before adding primary antibodies against alpha smooth muscle actin, collagen 1A1 in HCF and VE-Cadherin and ICAM in HUVEC. All antibodies were diluted in 5% donkey serum in PBS. The manufacturers and concentrations are indicated in table 3. The cells were incubated with primary antibodies over night at 4°C. After washing with PBS, secondary antibodies were added together with DAPI (1:1000, Merck) for one hour at room temperature. The manufacturers and concentrations are indicated in table 3. After another four washing steps, Fluoromount-G mounting medium was carefully added to each chamber.

Images were captured using a Leica STELLARIS 8 microscope. Signal quantification was performed using the Velocity software v7 (Quorum Technologies).

### Statistical Analysis

Normal distribution was tested by visualisation as frequency distribution histograms and Shapiro-Wilk tests. For normally distributed data with ≥ 3 samples, a two-way ANOVA with Dunnett's post-hoc testing was performed. For non-normally distributed data with ≥3 samples, a Kruskal-Wallis test with Dunn's post-hoc testing was performed. For pairwise comparisons of knockdown efficacy, a Mann-Whitney U test was performed between the scrambled control siRNA and the respective targeting siRNA. Graphs show mean ± SD unless otherwise indicated. The null hypothesis was rejected if p < 0.05. All statistical analyses were performed in Prism v10 (GraphPad).

**Table 2: Primer Sequences**

| **Gene name** | **Species** | **Forward** | **SEQ ID** | **Reverse** | **SEQ ID** |
|---|---|---|---|---|---|
| *RPLP0* | Homo sapiens | GGCGACCTGGA AGTCCAACT | 28 | CCATCAGCACCACA GCCTTC | 29 |
| *RPS4Y* | Homo sapiens | TGAGGAAGATTA CTGTGGGAGTG | 30 | AATCACCATACACAA ATTGCCTGT | 31 |
| *DDX3Y* | Homo sapiens | AAGCTTGTCAAA GGGATGAGTC | 32 | TCAGAGTTCAGGTC CAGATTAGC | 33 |
| *EIF1AY* | Homo sapiens | CATCTCCAGGAC CAAATGTGTCT | 34 | GGAAGCATTGTGTT TTGATGGTG | 35 |
| *USP9Y* | Homo sapiens | AGCAGTTGTCCT GTTGCTTACCA | 36 | GGGGCATCTTCTGA TGGAGAG | 37 |
| *ZFY* | Homo sapiens | GCAGGCGTGAG GAGCTG | 38 | GGCTCTTGTGGCTG CAATTC | 39 |
| *UTY* | Homo sapiens | TGTGCACGAAAA ACAAGCAAAAG | 40 | AGGATGATAATGAA AGAGCTAGTGT | 41 |
| *LINC00287* | Homo sapiens | CTACAACCACTC AAGGTCTGC | 42 | TATGACTGCTGTTGA GAGCG | 43 |
| *TTTY14* | Homo sapiens | GTGACGGATAAG GGGTGTGG | 44 | ATCTCGGTCCATCTC AGGGT | 45 |
| *KDM5D* | Homo sapiens | TTACTCCTCGCGT CCAAAG | 46 | ACGATCCTTGCAGA TGGCTT | 47 |
| *Nppa* | Rattus norvegicus | CCCGTATACAGTG CGGTGTC | 48 | TCAATCCTACCCCGA AGCA | 49 |
| *Acta1* | Rattus norvegicus | GCACCGCAAATG CTTCTAGG | 50 | CGATGGTCGATTGT CGTCCT | 51 |
| *Gapdh* | Rattus norvegicus | TCTCTGCTCCTCC CTGTTCTA | 52 | ATGAAGGGGTCGTT GATGGC | 53 |

**Table 3: Antibodies**

| Target | Manufacturer | Cataloque Number | Dilution |
|---|---|---|---|
| Collagen 1A1 | Cell Signaling | 72026 | 1:200 |
| Alpha Smooth Muscle Actin | Sigma Aldrich | C6168 | 1:200 |
| Phalloidin Alexa 488 | Sigma Aldrich | O7466 | 1:100 |
| ICAM-1 | Cell Siqnalinq | 62133 | 1:200 |
| VE-Cadherin | Cell Signaling | 2500 | 1:200 |
| Donkey anti-rabbit Alexa 555 | Thermo Fisher | A-24128 | 1:200 |
| Donkey anti-rabbit Alexa 647 | Thermo Fisher | A-31573 | 1:200 |
| Donkey anti-mouse Alexa 647 | Thermo Fisher | A-32787 | 1:200 |

### REFERENCES

The references are:
1. Li, H., M.H. Hastings, J. Rhee, L.E. Trager, J.D. Roh, and A. Rosenzweig, Targeting Age-Related Pathways in Heart Failure. Circ Res, 2020. 126(4): p. 533-551.
2. North, B.J. and D.A. Sinclair, The intersection between aging and cardiovascular disease. Circ Res, 2012. 110(8): p. 1097-108.
3. Kaur, G. and E. Lau, Sex differences in heart failure with preserved ejection fraction: From traditional risk factors to sex-specific risk factors. Womens Health (Lond), 2022. 18: p. 17455057221140209.
4. Forsberg, L.A., C. Rasi, N. Malmqvist, H. Davies, S. Pasupulati, G. Pakalapati, J. Sandgren, T. Diaz de Stahl, A. Zaghlool, V. Giedraitis, L. Lannfelt, J. Score, N.C. Cross, D. Absher, E.T. Janson, C.M. Lindgren, A.P. Morris, E. Ingelsson, L. Lind, and J.P. Dumanski, Mosaic loss of chromosome Y in peripheral blood is associated with shorter survival and higher risk of cancer. Nat Genet, 2014. 46(6): p. 624-8.
5. Haitjema, S., D. Kofink, J. van Setten, S.W. van der Laan, A.H. Schoneveld, J. Eales, M. Tomaszewski, S.C.A. de Jager, G. Pasterkamp, F.W. Asselbergs, and H.M. den Ruijter, Loss of Y Chromosome in Blood Is Associated With Major Cardiovascular Events During Follow-Up in Men After Carotid Endarterectomy. Circ Cardiovasc Genet, 2017. 10(4): p. e001544.
6. Zhou, W., M.J. Machiela, N.D. Freedman, N. Rothman, N. Malats, C. Dagnall, N. Caporaso, L.T. Teras, M.M. Gaudet, S.M. Gapstur, V.L. Stevens, K.B. Jacobs, J. Sampson, D. Albanes, S. Weinstein, J. Virtamo, S. Berndt, R.N. Hoover, A. Black, D. Silverman, J. Figueroa, M. Garcia-Closas, F.X. Real, J. Earl, G. Marenne, B. Rodriguez-Santiago, M. Karagas, A. Johnson, M. Schwenn, X. Wu, J. Gu, Y. Ye, A. Hutchinson, M. Tucker, L.A. Perez-Jurado, M. Dean, M. Yeager, and S.J. Chanock, Mosaic loss of chromosome Y is associated with common variation near TCL 1A. Nat Genet, 2016. 48(5): p. 563-8.
7. Thompson, D.J., G. Genovese, J. Halvardson, J.C. Ulirsch, D.J. Wright, C. Terao, O.B. Davidsson, F.R. Day, P. Sulem, Y. Jiang, M. Danielsson, H. Davies, J. Dennis, M.G. Dunlop, D.F. Easton, V.A. Fisher, F. Zink, R.S. Houlston, M. Ingelsson, S. Kar, N.D. Kerrison, B. Kinnersley, R.P. Kristjansson, P.J. Law, R. Li, C. Loveday, J. Mattisson, S.A. McCarroll, Y. Murakami, A. Murray, P. Olszewski, E. Rychlicka-Buniowska, R.A. Scott, U. Thorsteinsdottir, I. Tomlinson, B.T. Moghadam, C. Turnbull, N.J. Wareham, D.F. Gudbjartsson, C. International Lung Cancer, C. Breast Cancer Association, B. Consortium of Investigators of Modifiers of, C. Endometrial Cancer Association, C. Ovarian Cancer Association, C. Prostate Cancer Association Group to Investigate Cancer Associated Alterations in the Genome, G.M.-A.P. Kidney Cancer, Q.C. e, C. Biobank-based Integrative Omics Study, T. andMe Research, Y. Kamatani, E.R. Hoffmann, S.P. Jackson, K. Stefansson, A. Auton, K.K. Ong, M.J. Machiela, P.R. Loh, J.P. Dumanski, S.J. Chanock, L.A. Forsberg, and J.R.B. Perry, Genetic predisposition to mosaic Y chromosome loss in blood. Nature, 2019. 575(7784): p. 652-657.
8. Forsberg, L.A., J. Halvardson, E. Rychlicka-Buniowska, M. Danielsson, B.T. Moghadam, J. Mattisson, C. Rasi, H. Davies, L. Lind, V. Giedraitis, L. Lannfelt, L. Kilander, M. Ingelsson, and J.P. Dumanski, Mosaic loss of chromosome Y in leukocytes matters. Nat Genet, 2019. 51(1): p. 4-7.
9. Mas-Peiro, S., W.T. Abplanalp, T. Rasper, A. Berkowitsch, D.M. Leistner, S. Dimmeler, and A.M. Zeiher, Mosaic loss of Y chromosome in monocytes is associated with lower survival after transcatheter aortic valve replacement. Eur Heart J, 2023. 44(21): p. 1943-1952.
10. Sano, S., K. Horitani, H. Ogawa, J. Halvardson, N.W. Chavkin, Y. Wang, M. Sano, J. Mattisson, A. Hata, M. Danielsson, E. Miura-Yura, A. Zaghlool, M.A. Evans, T. Fall, H.N. De Hoyos, J. Sundstrom, Y. Yura, A. Kour, Y. Arai, M.C. Thel, Y. Arai, J.C. Mychaleckyj, K.K. Hirschi, L.A. Forsberg, and K. Walsh, Hematopoietic loss of Y chromosome leads to cardiac fibrosis and heart failure mortality. Science, 2022. 377(6603): p. 292-297.
11. Charchar, F.J., L.D. Bloomer, T.A. Barnes, M.J. Cowley, C.P. Nelson, Y. Wang, M. Denniff, R. Debiec, P. Christofidou, S. Nankervis, A.F. Dominiczak, A. Bani-Mustafa, A.J. Balmforth, A.S. Hall, J. Erdmann, F. Cambien, P. Deloukas, C. Hengstenberg, C. Packard, H. Schunkert, W.H. Ouwehand, I. Ford, A.H. Goodall, M.A. Jobling, N.J. Samani, and M. Tomaszewski, Inheritance of coronary artery disease in men: an analysis of the role of the Y chromosome. Lancet, 2012. 379(9819): p. 915-922.
12. Bloomer, L.D., C.P. Nelson, J. Eales, M. Denniff, P. Christofidou, R. Debiec, J. Moore, C. Cardiogenics, E. Zukowska-Szczechowska, A.H. Goodall, J. Thompson, N.J. Samani, F.J. Charchar, and M. Tomaszewski, Male-specific region of the Y chromosome and cardiovascular risk: phylogenetic analysis and gene expression studies. Arterioscler Thromb Vasc Biol, 2013. 33(7): p. 1722-7.
13. Wu, S., L. Zhang, J. Deng, B. Guo, F. Li, Y. Wang, R. Wu, S. Zhang, J. Lu, and Y. Zhou, A Novel Micropeptide Encoded by Y-Linked LINC00278 Links Cigarette Smoking and AR Signaling in Male Esophageal Squamous Cell Carcinoma. Cancer Res, 2020. 80(13): p. 2790-2803.

## Claims

1. **A compound or composition for use in the treatment of a cardiovascular disease** in a subject, wherein the compound or composition is, or comprises, an agonist of expression, function and/or stability of a gene, or protein product of a gene, selected from the non-coding genes and *Long Intergenic Non-Coding RNA 278* (*LINC00278*) and *Testis Expressed Transcript Y-linked 14 (TTTY14)* and the further genes *Ribosomal Protein S4 Y-Linked 1* (*RPS4Y1*)*, DEAD-Box Helicase 3 Y-linked* (*DDX3Y*), *Eukaryotic Translation Initiation Factor 1A Y-linked* (*EIF1AY*)*, Ubiquitously Transcribed Tetratricopeptide Repeat Containing Y-linked (UTY), Zink Finger Protein Y-linked (ZFY), Lysine Demethylase 5D (KDM5D),* and *Ubiquitin Specific Peptidase 9 Y-linked* (*USP9Y*).

2. The compound or composition for use of claim 1, wherein the compound or composition is an agonist of the expression of a protein product of the gene, preferably the agonist is protein comprising the sequence of the protein product of the gene, or is a nucleic acid construct for the expression of the protein product of the gene.

3. The compound or composition of any one of claims 1 or 2, where the agonist is a YY1-blocking micropeptide.

4. The compound or composition for use of any one of claims 1 to 3, wherein the treatment involves administration of a genetically modified cell to the subject, wherein the genetically modified cell is modified to express one or more of the genes.

5. **A method for identifying** a **modulator cell-mediated regulation of myocardial cell function,** the method comprising the steps:
**(a)** Providing a first cell and a second cell, wherein the second cell is a myocardial cell;
**(b)** Culturing the first cell to obtain a test first cell under conditions which are selected from:
(i) where the expression one or more known candidate genes are being perturbed, preferably knocked out or knocked in, in the first cell:
(ii) With one or more genetic expression constructs being expressed in the first cell to overexpress, or ectopically express, one or more known candidate genes in the first cell; and
(iii) Where the first cell is cultured in contact with one or more candidate compounds
**(c)** optionally culturing a control first cell under control conditions (not the conditions of step (b));
**(d)** Bringing into contact the second cell and the test first cell, or the second cell and conditioned medium of the culture of the test first cell, and, optionally, culturing the second cell under the contact conditions:
Wherein a difference of the test first cell to a control or control first cell in one or more markers of myocardial function indicates that the one or more known candidate genes or compounds is a modulator of a cell mediated regulation of myocardial cell function.

6. The method of claim 5, wherein the marker of myocardial function is selected from beating frequencies of cardiomyocytes, established protein markers of fibroblast activation or expression of known cell-cell interaction protein.

7. The method of claim 5 or 6, wherein the first cell is an immune cell such as a PBMC, a PBMC-derived or THP-1 monocyte, in particular a macrophage such as an M0 macrophage.

8. The method of any one of claims 5 to 7, wherein the second cell is selected from primary human cardiac fibroblasts (HCF), neonatal rat cardiomyocytes (NRCM) and human umbilical vein endothelial cells (HUVEC).

9. The method of any one of claims 5 to 8, wherein the known candidate gene is a Y-chromosome located gene.

10. **A method for the identification of a compound** useful in the treatment or prevention of a cardiovascular disorder, the method comprising the step of:
**(a)** Providing a candidate compound, composition, or a candidate medical procedure;
**(b)** Providing a test cell or test tissue, wherein the test cell or test tissue is involved with or is associated with the cardiovascular disease;
**(c)** Contacting the test cell or test tissue with the candidate compound, composition or performing on the test cell or test tissue the candidate medical procedure;
**(d)** Determining in the test cell or test tissue a test gene expression level, or a level of protein, wherein the protein is expressed by such test gene, the test gene being selected from the group consisting of *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDMSD), Ubiquitin Specific Peptidase 9 Y-linked (USP9Y) and the non-coding genes Testis Expressed Transcript Y-linked 14 (TTTY14)* and *Long Intergenic Non-Coding RNA 278 (LINC00278);*
Wherein an increased gene expression level, or an increased protein level in the test cell or test tissue compared to a reference, or compared to a control cell or control tissue not contacted with the candidate compound or composition or medical procedure, indicates that the respective candidate compound or composition or candidate medical procedure is useful in the treatment or prevention of a cardiovascular disease.

11. The method of claim 10, wherein the candidate compound or composition comprises a nucleic acid construct for the expression of protein or peptide sequence, wherein the protein or peptide sequence comprises an amino acid sequence, or a fragment thereof, encoded by any one of the test genes.

12. The method of claim 10, wherein the candidate compound or composition comprises a protein or peptide, wherein the protein or peptide comprises an amino acid sequence, or a fragment thereof, encoded by any one of the test genes.

13. **A method for diagnosing a subject,** the method comprising determining in a biological sample of the subject the level of gene or protein expression of at least one biomarker selected from *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDMSD), Ubiquitin Specific Peptidase 9 Y-linked (USP9Y) and the non-coding genes Testis Expressed Transcript Y-linked 14 (TTTY14)* and *Long Intergenic Non-Coding RNA 278 (LINC00278);* wherein a reduced gene or protein expression indicates an increased risk of cardiovascular disease in the subject.

14. **A use of a biomarker** for the diagnosis of an increased risk of cardiovascular disease in a subject, wherein the protein expression or gene expression is selected from an expression of the genes *Ribosomal Protein S4 Y-Linked 1 (RPS4Y1), DEAD-Box Helicase 3 Y-linked (DDX3Y), Eukaryotic Translation Initiation Factor 1A Y-linked (EIF1AY), Ubiquitously Transcribed Tetratricopeptide Repeat Containing Y-linked (UTY), Zink Finger Protein Y-linked (ZFY), Lysine Demethylase 5D (KDM5D), Ubiquitin Specific Peptidase 9 Y-linked (USP9Y) and the non-coding genes Testis Expressed Transcript Y-linked 14 (TTTY14)* or *Long Intergenic Non-Coding RNA 278 (LINC00278).*

15. The method of any one of the preceding claims, wherein the gene or test gene is selected from *UTY, ZFY, TTTY14* and *LINC00278,* and preferably is *LINC00278.*
